# EUROPEAN PATENT APPLICATION

(11) **EP 4 445 926 A1**
(43) Date of publication of application: **16.10.2024**
(21) Application number: 23167948.1
(22) Date of filing: 14.04.2023
(51) Int. Cl.: A61M 1/16

(54) **SYSTEM AND METHOD FOR DETECTING A CONCENTRATE CONTAINER CONNECTED TO A BLOOD TREATMENT DEVICE**

(71) Applicant: Fresenius Medical Care Deutschland GmbH, 61352 Bad Homburg (DE)
(72) Inventor: WOHLFEIL, Florian, 61352 Bad Homburg (DE); GAGEL, Alfred, 61352 Bad Homburg (DE); PFEIFER, Sebastian, 61352 Bad Homburg (DE); LAFFAY, Philippe, 61352 Bad Homburg (DE)
(74) Representative: Herrmann, Uwe

(57) **Abstract**

The present invention relates to a blood treatment device, comprising a housing, a connection means configured to fluidically and / or mechanically connect the blood treatment device to a container, wherein the container comprises a closing element fluidically closing the container against the outside, and at least one sensor configured to detect the presence of a container connected to the connection means and further configured to detect an integrity status of the closing element of the container.

## Description

The present invention refers to a system and a method for detecting a concentrate container connected a blood treatment device, preferably a dialysis machine for renal replacement therapy, and for determining the state (intact or broken) of a closing element fluidically closing the container. Further aspects of the present invention refer to a container and a blood treatment device configured to be used in a method according to the present invention.

For chronic blood treatment, especially for chronic dialysis, nowadays almost exclusively bicarbonate-based dialysis solutions are used, that are generated by the blood treatment device itself / online during treatment by diluting an acidic and a basic concentrate, preferably a powder concentrate, with water. Thus, it is established practice that the basic concentrate is generated online during the treatment, i.e. that liquid basic concentrate is continuously generated during the treatment by diluting a dry concentrate/powder concentrate with water.

For this purpose, dry powder concentrate is generally provided in a container that is fluidic ally coupled to the blood treatment device so that water can be dispensed into the container by the blood treatment device for dissolving the dry concentrate. Simultaneously the basic liquid concentrate / the bicarbonate solution generated in this way is withdrawn by the blood treatment device from the concentrate container.

When using such containers for the preparation of dialysis solutions it is essential to ensure that the correct container comprising the correct contents is connected to the blood treatment device, the container is properly sealed prior to connection to the blood treatment device to ensure purity of the contents and the seal of the container is correctly broken by the blood treatment device to fluidically couple the container to the blood treatment device.

In order to prevent contaminations of the dialysis fluid to be used, it is important to verify that a closing element fluidically sealing a container is in an intact state before the container is fluidically coupled to the blood treatment device by opening or breaking the closing element.

Conventionally, the container and its closing elements are thus manually inspected, e.g. by a nurse, which poses a further burden on medical personnel.

Against this background it is an object of the present invention to alleviate or even completely abolish the disadvantages of the prior art. In particular, it is an object of the present invention to provide a container and a blood treatment device that allows for an easy and reliable detecting of the presence of a container attached to the blood treatment device and / or a state of integrity of a closing element of the container.

This object is solved by the present invention, in particular by a container and blood treatment device comprising the features recited in the independent claims.

An aspect of the present invention relates to a blood treatment device, comprising a housing, a connection means configured to fluidically and / or mechanically connect the blood treatment device to a container, wherein the container comprises a closing element fluidically closing the container against the outside, and at least one sensor configured to detect the presence of a container connected to the connection means and further configured to detect an integrity status of the closing element of the container.

The connection means can be understood as that section of the blood treatment device configured to connect to a container and can comprise at least one attachment element and / or connection element.

One single sensor can be configured to detect the presence of a container connected to the connection means and can be further configured to detect an integrity status of the closing element of the container.

The sensor can be an optical sensor. The blood treatment device can further comprise at least one inspection window configured to allow the optical sensor a field of inspection of the closing element of the container, in a position in that the container is connected to the blood treatment device, wherein the inspection window preferably comprises a transparent section preferably comprising a polymer material.

The sensor can be an optical sensor detecting in the red, green, blue and optionally clear channels. The sensor can comprise a proximity sensor, e.g. an infra-red (IR) proximity sensor. The proximity sensor can be used to detect the presence of a container attached to the blood treatment device.

For example, the inspection window can be an opening in a wall through that the optical sensor can inspect the closing element of the container.

The opening can be closed by an optional transparent section to prevent contaminations accumulating in the opening or in the interior of the blood treatment device and to protect the sensor against damage.

The blood treatment device further can comprise at least one illumination window configured to allow a light source of the blood treatment device to illuminate the closing element of the container and / or at least a part of the container, in a position in that the container is connected to the blood treatment device. For purposes of illumination, the blood treatment device can comprise a light source emitting light in the red, green, blue, clear (white) and infra-red spectrum. For example, in case of the IR proximity sensor detecting the presence of a container attached to the blood treatment device, the sensor can be configured to activate the light source to emit clear / white light. This increases detection robustness.

The illumination window preferably can be arranged adjacent to the inspection window and can comprise a transparent section preferably comprising a polymer material.

The inspection window and the illumination window or windows can be realised as separate windows each corresponding to a separate opening or as a common or joint window formed for example as a single opening in a wall.

In the latter case, a light source of the blood treatment device can illuminate the closing element of the container through the same opening or window through that the optical sensor can inspect the closing element of the container.

The term "window" can be understood to mean an opening with or without a transparent section or cover preferably made of polymer material.

In a case in that the inspection window and the illumination window are realised as two separate windows, the inspection window and the illumination window can be closed or covered by a common or joint transparent section or can each comprise a separate transparent section.

The inspection window can be arranged such that the closing element of the container is at least partially present in the field of inspection in a state in that the closing element is intact and in a state in that the closing element is opened. In other words, according to an embodiment, the optical sensor can inspect the closing element of a container connected to the blood treatment device in any integrity status ranging from intact to fully broken. In addition to that, the presence or absence of the closing element can be detected.

A blood treatment device according to the present disclosure, can further comprise an evaluation unit configured to determine based on optical measurement data from the optical sensor a proportion of the field of vision in that the closing element of the container is present and derive therefrom a conclusion regarding the presence of the container at the blood treatment device and / or a conclusion regarding an integrity status of the closing element, wherein the integrity status is preferably selected from the group consisting of intact/closed, partially opened and opened.

The evaluation unit and / or control unit can be a processor, such as a digital signal processor (DSPs), general purpose microprocessor, application specific integrated circuit (ASICs), field programmable logic array (FPGAs), or other equivalent integrated or discrete logic circuitry. Accordingly, the terms "evaluation unit" and "control unit" as used herein may refer to any of the foregoing structure or any other physical structure suitable for implementation of the described techniques.

The same holds try for the memory unit that can be any physical structure suitable for storing electronic data, such as a computer-readable medium. Computer-readable media may include non-transitory computer-readable media, which corresponds to a tangible medium such as data storage media (e.g., RAM, ROM, EEPROM, flash memory, or any other medium that can be used to store desired program code in the form of instructions or data structures and that can be accessed by a computer.

If in the present disclosure it is recited that a unit, such as the evaluation unit, control unit or memory unit, is configured to perform a given step, this can be understood as the unit specifically being programmed to perform the given step.

Further, a method according to the present invention for connecting a container for concentrate to a blood treatment device, can comprise the steps: determining based on optical measurement data from the optical sensor a proportion of the field of vision in that the closing element of the container is present; and deriving therefrom a conclusion regarding the presence of the container at the blood treatment device and / or a conclusion regarding an integrity status of the closing element, wherein the integrity status is preferably selected from the group consisting of intact/closed, partially opened and opened. Alternatively or additionally, the presence of the container at the blood treatment device can be detected by means of an IR proximity sensor integrated in the optical sensor detecting in the red, green, blue and optionally clear channels.

The optical sensor of a blood treatment device according to the present invention can be a colour sensor configured to measure optical intensities in at least three channels and the blood treatment device preferably comprises an evaluation unit configured to determine a colour of a closing element of a container.

Further, a method according to the present disclosure can comprise the step of determining a colour of a closing element of a container connected to the blood treatment device.

A blood treatment device according to the present disclosure can comprise a memory in that optical characteristics, in particular characteristic optical intensities in at least three channels, of different types of containers connectable to the blood treatment device and / or of closing elements of such containers are stored.

The evaluation unit can be configured to retrieve from the memory based on a determined colour of the closing element of the container a corresponding type of the container, in particular a corresponding size or volume or amount of content contained in the container.

Further, a method according to the present disclosure can comprise the step of retrieving from a memory based on a determined colour of a closing element of a container a corresponding type of container, in particular a corresponding size or volume or amount of content contained in the container.

In the memory of a blood treatment device according to the present disclosure for each type of container at least one set of upper and lower limit values for each channel of the at least three channels can be stored.

For each type of container one set of upper and lower limit values for each of the at least three channels corresponding to an intact closing element and / or one set of upper and lower limit values for each of the at least three channels corresponding to an opened closing element and / or one set of upper and lower limit values for each of the at least three channels corresponding to container without a closing element can stored in the memory.

The at least one sensor of a blood treatment device according to the present invention can be a force sensor, preferably a strain gauge, configured to measure a force required to break a closing element present at a container upon connection of the container to the blood treatment device and / or configured to measure a force exerted by a container connected to the connections means od the blood treatment device.

In principle, blood treatment device according to the present disclosure can comprise one or more optical sensors and one or more force sensors.

The at least one force sensor can located at a lever at that a machine connection element configured to fluidically connect the blood treatment device to the container is pivotably arranged, wherein the lever is pivotably arranged at the housing.

Such a force sensor can serve the purpose of determining a force required to open a closing element closing a container.

Additionally or alternatively, at least one force sensor can be located at an attachment element comprised by the connection means, wherein the attachment means is configured to hold a container in a position in that the container is attached to the blood treatment device and / or in that the container is connected to the blood treatment device via the connection means.

Such a force sensor can serve the purpose of determining a force exerted onto the attachment element and / or connection means of the blood treatment device when a container is attached and / or connected to the blood treatment device.

In case of the blood treatment device comprising two machine attachment elements and the container comprising two container attachment elements, it can be verified whether an equal amount of force is exerted by the two container attachment elements on the two machine attachment elements. An equal or uniform exertion of force can imply a correct attachment of the container to the blood treatment device. Thus, the equal distribution of force exerted onto the attachment means can be used to check for a correct attachment of a container with two container attachment elements.

A blood treatment device according to the present disclosure can comprise an evaluation unit configured to derive from measurement data of at least one force sensor a conclusion regarding the integrity status of the closing element, wherein the integrity status is preferably selected from the group consisting of intact/closed, partially opened and opened.

Further, a method according to the present disclosure can comprise the step of deriving from measurement data of at least one force sensor a conclusion regarding the integrity status of a closing element, preferably a closing element of a container attached and / or connected to the blood treatment device, wherein the integrity status is preferably selected from the group consisting of intact/closed, partially opened and opened.

In a memory of a blood treatment device according to the present disclosure, force measurement values can be stored corresponding to an intact closing element and / or a partially opened and / or a broken closing element.

A blood treatment device according to the present disclosure can comprise an evaluation unit configured to retrieve from a memory of the blood treatment device based on the measurement data of at least one force sensor, a type, in particular a weight, size, volume or amount of content of a container attached to the blood treatment device.

Further, a method according to the present disclosure can comprise the step of retrieving from a memory of the blood treatment device based on the measurement data of at least one force sensor, a type, in particular a weight, size, volume or amount of content of a container attached to the blood treatment device.

It should be noted that the functionalities disclosed above with regard to the evaluation unit can be implemented in a single evaluation unit comprising all these functionalities or multiple separate evaluation units can execute these functionalities. The same holds true for the memory.

Another aspect of the present disclosure pertains to a container for concentrate, comprising:
a hollow body for containing concentrate and a container connection element configured to fluidically and / or mechanically connect the container to a blood treatment device, wherein the container connection element comprises an inspection window configured to allow an optical sensor of a blood treatment device in a field of inspection an optical inspection of a closing element, in particular a cap, fluidically closing the container, in an position in that the container is connected to the blood treatment device.

The inspection window can be implemented as an opening in a wall that can optionally be covered or closed by a transparent section.

The opening in the wall forming the inspection window can have a triangular shape. This shape is particularly suitable if the blood treatment device comprises an optical sensor and two light sources, such as LEDs, that are arranged to form a triangle. Thus, the triangular shape of the inspection window preferably matches the triangular arrangement of the sensor and light source(s). This arrangement allows for an optimal illumination while reducing the overall size of the inspection window. Reducing the overall size of the inspection window increases the overall stability of the wall in that the inspection window is present.

The connection element of a container according to the present disclosure can comprise an outer tube and an inner tube preferably arranged concentrically to the outer tube. The inspection window can be formed as an opening in the outer tube arranged so that through the inspection window a closing element present on the inner tube can be inspected. This is achieved for example by arranging the opening in the outer wall forming the inspection window such that the closing element arranged on the inner tube is at least partially in the same plane as the window. An outer surface of the inner tube can have a polygonal, preferably a hexagonal, geometry.

On the inner tube of the connection element a closing element, preferably a cap, can be arranged and the inspection window can be arranged so that the closing element is at least partially in the field of inspection in a state in that the closing element is intact and / or in a state in that the closing element is opened. An inner contour of the closing element arranged on the inner tube can be configured to match the polygonal, preferably hexagonal, geometry of the outer surface of the inner tube.

Another aspect of the present disclosure pertains to a system comprising at least one blood treatment device, preferably a blood treatment device according to the present disclosure, and at least one container, preferably a container according to the present disclosure, wherein the container is reversibly connectable to the blood treatment device by means of a container connection element present on the container interacting with connection means present on the blood treatment device in a position in that the inspection window of the container and the inspection window of the blood treatment device are at least partially aligned to allow at least one optical sensor of the blood treatment device to optically inspect at least one closing element of the container.

Yet another aspect of the present disclosure pertains to a method for connecting a container for concentrate, preferably a container according to the present disclosure, to a blood treatment device, preferably a blood treatment device according to the present disclosure, comprising the step:
Detecting the presence and / or integrity of the container, in particular a closing element or breakable cap thereof, connected to the blood treatment device by means of at least one optical sensor and / or force sensor present on or associated with the connections means of the blood treatment device.

A method according to the present disclosure can further comprise one or more of the method steps disclosed above e.g. in the context of the evaluation unit.

In other words, aspects of the present invention can be described as follows:
A blood treatment device according to the present invention comprises a main body or housing,
two machine attachment elements to that corresponding container attachment elements of a container for concentrate can be attached, wherein the two machine attachment elements project in a direction projecting essentially at right angles from a side surface of the main body or surface of the housing, and two machine connection elements configured to fluidically couple the blood treatment device to a container for concentrate, wherein the blood treatment device comprises a sensor, preferably an optical sensor or force sensor, configured to detected the presence and / or integrity of a container and / or of a closing element fluidically closing the container connected to the blood treatment device.

In other words, the blood treatment device is equipped with at least one sensor configured to detect whether a container has been attached to the blood treatment device and whether a closing element or cap fluidically closing the container is intact or not.

An optical sensor can be used for this purpose. For example, according to an embodiment, the two attachment elements can each comprise a protrusion, preferably formed as a stud, and an optical sensor is arranged in the vicinity of a stud behind an inspection window in an outer contour of the blood treatment device, so that the optical sensor can detect through the inspection window whether a container is attached to the attachment elements of the blood treatment device.

The inspection window is preferably configured to allow the optical sensor an unobstructed field of inspection of a closing element of the container, with the inspection window preferably being arranged such that the closing element of the container is at least partially present in the field of inspection in a state in that the closing element is intact and in a state in that the closing element is opened.

As the optical sensor is arranged inside of the blood treatment device and views the outside via the inspection window, the sensor is protected from damage e.g. by the container that is attached to the blood treatment device.

The blood treatment device can further comprise an evaluation unit configured to determine based on optical measurement data from the optical sensor a proportion of the field of vision in that the closing element of the container is present and derive therefrom a conclusion regarding the presence of the container at the blood treatment device and / or a conclusion regarding an integrity of the closing element.

For example, the sensor can comprise a photoreflective element to determine whether a container has been attached to the blood treatment device.

Alternatively or additionally, a blood treatment device according to the present disclosure can comprise a photoreflective element as a component of an optical sensor. The photoreflective element can be configured to illuminate a container or closing element thereof and detect light reflected by the container or closing element thereof. The distance the light travels from the closing element back to the sensor can indicate an integrity status of the closing element. For example, if the cap of the container is closed, light reflected by the cap travels a given distance x. If the cap is opened / broken, broken components of the cap are arranged closer to the sensor and the light reflected by the cap travels a shorter distance x-y.

Determining the proportion of the field of vision in that the closing element of the container is present allows to determine whether the closing element is closed / intact or broken / open. For example, in the intact state, the closing element may make up a first proportion of the field of vision or inspection by the sensor. As the closing elements is broken by the connection elements of the blood treatment device to fluidically couple the container to the blood treatment device, the position of the closing element or at least some elements thereof is changed. The closing element may thus make up a second proportion of the field of vision or inspection by the sensor that is larger or smaller than the first portion.

The blood treatment device can comprise a memory in that a relationship is stored between the proportion of the field of vision in that the closing element of the container is present and the integrity state (intact, partially opened, fully opened) of the closing element. An evaluation unit can be provided that is configured to retrieve from the memory a corresponding integrity state of the closing element based on a detected proportion of the field of vision in that the closing element of the container is present.

According to an embodiment, the optical sensor is a colour sensor (in particular an RGB sensor) configured to measure optical intensities in red, green, blue and optionally clear channels and the blood treatment device comprises an evaluation unit configured to determine a colour of a closing element of a container. The clear channel can also be used for calibration.

This offers the opportunity to colour code the closing elements (e.g. caps) of the container based e.g. on the content of the container, the size of the container or any other desirable parameter of the container.

For example, a green cap can indicate a concentrate of a first type being contained in the container and a blue cap can indicate a concentrate of a second type being contained in the container. Similarly, the colour of the caps can indicate the size or volume of the bag with e.g. a blue cap indicating a bag of 250 ml volume and a green cap indicating a bag of 100 ml volume.

Preferably, the cap or caps of the container are coloured in red, green or blue, as these colours have proven to be reliably detectable by an optical sensor. The colouring of the cap thus is not only of aesthetic relevance, but also of technical relevance in the interaction with the sensor.

A blood treatment according to the invention can comprise a memory in that optical characteristics, in particular characteristic optical intensities in at least red, green, blue and optionally clear channels, of different types of containers connectable to the blood treatment device are stored, wherein the evaluation is configured to retrieve from the memory based on a determined colour of the closing element of the container a corresponding type of the container, in particular a corresponding size or volume of the container.

This allows the blood treatment device to determine the type of container attached to the device based on the measurement results of the optical sensor.

The blood treatment device may be configured to display the result of the evaluation unit, e.g. "250 ml- bag connected" to user via a user interface such as a screen.

Also, the blood treatment device can be configured to perform a plausibility check to determine whether the identified type of bag (e.g. a 250 ml bag) is suitable for a treatment to be performed by the blood treatment device (e.g. a treatment requiring 2L of solution) and notify or alert a user accordingly if a mismatch has been detected.

Apart from determining the type of container, the optical sensor can be used to determine an integrity state of at least one closing element / cap fluidically closing the container.

For example, in the memory of the blood treatment device for each type of container at least one set of upper and lower limit values for each channel of the red, green, blue and optionally clear channels can be stored, wherein preferably for each type of container one set of upper and lower limit values for each channel of the red, green, blue and optionally clear channels corresponding to an intact closing element and one set of upper and lower limit values for each channel of the red, green, blue and optionally clear channels corresponding to an opened closing element are stored.

For example, for a given bag (e.g. a 250 ml bag comprising a green cap) characteristic intensity values (in the red, blue, green and optional clear channels) representative of an intact state of the closing element and an opened state of the closing element can be stored in the memory of the blood treatment device as illustrated below:

| 1. Intact | | 2. opened | |
|---|---|---|---|
| R_lower_1 | R_upper_1 | R_lower_2 | R_upper_2 |
| G_lower_1 | G_upper_1 | G_lower_2 | G_upper_2 |
| B_lower_1 | B_upper_1 | B_lower_2 | B_upper_2 |
| C_lower_1 | C_upper_1 | C_lower_2 | C_upper_2 |

If for example, the detected intensity values fall within the upper and lower limit values representative of an intact state of the closing elements (two left columns in the chart above), an evaluation unit of the blood treatment device can conclude that the closing element of the container is intact. If for example, the detected intensity values fall within the upper and lower limit values representative of an opened state of the closing elements (two left columns in the chart above), an evaluation unit of the blood treatment device can conclude that the closing element of the container is broken / opened.

The status of the container and / or its closing element(s) can be conveyed to the user based on the optical measurement values and upper and lower limit values as follows.

If it has been verified by the evaluation unit of the blood treatment device that the connection elements of the blood treatment device have not yet been connected to the connection elements of the container, the status of the container can be given as follows:
1) "NEW", when the R, G, B, C measurement values fall within the 1. set of limit values indicating an intact closing element,
2) "USED", when the R, G, B, C measurement values fall within the 2. set of limit values indicating an broken closing element,
3) "UNKNOWN", when the R, G, B, C measurement values match none setting of limit data.
4) "No CAP", when the R, G, B, C measurement values fail to fall into the 1. or 2. set of limit values but fall within a 3. set of limit values indicating a connection element of a container without a closing element.

For example, the evaluation unit of the blood treatment device can be configured to verify that the machine connection elements of the blood treatment device have not yet been connected to the container connection elements of the container by detecting the position of a flap at that the machine connection elements are mounted. The position of the flap can be detected by means of a hall sensor. Alternatively, the evaluation unit can detect the force that the machine connection elements have exerted, because a higher force is required to break a closing element of the container and connect the machine connection elements to the container connection elements.

Thus, preferably prior to connecting the connection elements of the blood treatment device to the connection elements of the container, a user receives an indication whether the closing element of the container attached to the blood treatment device is intact or broken.

Then, the connection elements of the blood treatment device are connected to the connection elements of the container. The container and / or its closing element(s) can be set to "UNKNOWN", when the R, G, B, C measurement values determined after the connection of the container to the blood treatment device do not fall within the 2. set of limit values indicating a broken closing element. Thus, if the connection process is not performed properly or the closing element is not adequately broken, the user may be notified by the status of the container communicated to the user.

Furthermore, the integrity of the closing element(s) of a container can be determined not only with at least one optical sensor by also with a least one force sensor. The use of force sensors in the context of the present invention is further described below:
For example, force sensors can be arranged at at least one connection element of the blood treatment device to measure the force required to be exerted by the connection element(s) of the blood treatment device onto a closing element or closing elements of the container in order to open the closing element(s) and fluidically connect the container to the blood treatment device. This is based on the general fact that it takes less force to break an already opened or partially opened closing element or connect without the presence of a closing element than it takes to open an intact closing element.

According to an embodiment, the two connection elements of the blood treatment are pivotably arranged at a lever rotatably arranged at the blood treatment device, so that the two connection elements are smoothly movable from a movement along a circular path to an axial movement to insert the two connection elements of the blood treatment device into two corresponding connection elements of a container, preferably by axially moving the two connection elements of the blood treatment parallel to a longitudinal direction of the main body of the blood treatment device, wherein the two connection elements each comprise a force sensor, preferably a strain gauge, configured to measure the force required to break a closing element or closing elements present at a container upon connection of the container to the blood treatment device.

The force sensors can be each located at or adjacent to a connection point in that each connection element is pivotably arranged at the lever. The force sensors can also be arranged on or at the lever. In this position, the force sensors are well placed to measure the force exerted by the connection elements of the blood treatment device on corresponding closing elements of the container.

The blood treatment can comprise an evaluation unit configured to derive from measurement data of the force sensor(s) a conclusion regarding the integrity or absence of the closing element(s).

Further, the blood treatment can comprise a memory in that force measurement values are stored for an intact closing element and / a broken closing element.

For example, in the memory it can be stored that an intact closing element requires a force of around 40 N to be opened. If there are two closing elements in a container, the force required for opening the closing elements of the container sums up to roughly 80 N.

An already broken closing element requires a much lower force as only friction has to be overcome. For example, the force required to connect a container with an already opened closing element would require ca. 15 to 20 N per closing element / cap summing up to 30 - 40 N in total if two closing elements are present. Therefore, a differentiation between an intact and a broken/opened closing element or container can easily be made based on these differing values.

Furthermore, force sensors may also be used to detect the presence of a container attached to the attachment elements of the blood treatment device.

For this purpose, the two attachment elements of the blood treatment device can each comprise comprises a force sensor, preferably a strain gauge, configured to measure a force generated by the corresponding attachment element of the container placed on each attachment element of the blood treatment device.

For example, each attachment element of the blood treatment device can comprise a protrusion onto that a corresponding attachment element of the container can be placed, and each protrusion preferably comprises a force sensor, preferably a strain gauge, configured to measure a force generated by the corresponding attachment element of the container placed on the protrusion.

The blood treatment device may be equipped with a memory and an evaluation unit configured to retrieve from the memory of the blood treatment device based on the measurement data of the force sensors, a type, in particular a weight, size or volume, of a container attached to the blood treatment device. In case different forces at the two attachment elements are measured a warning about an incorrect container attachment can be generated.

Another aspect of the present invention relates to a container configured to be used with a blood treatment device according to the present invention.

Such a container for concentrate comprises a main body for containing concentrate, a connection portion having a flat end surface and configured to connect the container to a blood treatment device, two attachment elements arranged at two opposing sides of the connection portion and configured to reversibly attach the container to two corresponding attachment elements present on a blood treatment device, and
two connection elements each comprising an opening at the flat end surface of the connection portion and configured to interact with two corresponding connection elements present on a blood treatment device to fluidically couple the container with the blood treatment device by insertion of the connection elements present on the blood treatment device into the connection elements of the container, wherein at least one connection element comprises an inspection window allowing an optical sensor of a blood treatment device in a field of inspection an optical inspection of a closing element, in particular a cap, fluidically closing the container.

In other words, through the inspection window in the connection element of the container the optical sensor can inspect a closing element present in the connection element of the container in a field of vision / inspection defined by the inspection window.

For example, each connection element of the container comprises an outer tube and an inner tube preferably arranged concentrically to the outer tube and the inspection window is formed as an opening in the outer tube. Through the inspection window, a closing element arranged on the inner tube of the connection element can be inspected by an optical sensor arranged outside of the outer tube of the connection element or even outside of the container, e.g. being part of a blood treatment device.

An outer surface of the inner tube preferably has a polygonal, preferably a hexagonal, geometry that is mirrored by the geometry of an inner surface of a main body of the closing element into that the inner tube preferably is inserted. The hexagonal geometry aids opening of the closing element.

In a container according to an embodiment, on the inner tube of each connection element a closing element, preferably a cap, is arranged and the inspection window is arranged such that the closing element is at least partially in the field of inspection (also referred to as field of vision) in a state in that the closing element is intact and / or in a state in that the closing element is opened, wherein preferably an inner contour of the closing element arranged on the inner tube matches the polygonal, preferably hexagonal, geometry of the outer surface of the inner tube.

In other words, the inspection window is arranged such that the closing element is at least partially present in the field of inspection regardless of whether the closing element is intact or partially or completely opened.

Another aspect of the invention pertains to a system comprising at least one blood treatment device according to the present invention and at least one container according to the present invention, wherein preferably the container is reversibly attached to the blood treatment device by means of the two attachment elements present on the container interacting with the two attachment elements present on the blood treatment device and / or the two connection elements of the blood treatment device are fluidically coupled to the two connection elements of the container, and the inspection window of the container and the inspection window of the blood treatment device are at least partially aligned to allow at least one optical sensor of the blood treatment device to optically inspect at least one closing element of the container.

Yet another aspect of the invention pertains to a method for connecting a container for concentrate, preferably a container according to the present invention, to a blood treatment device, preferably a blood treatment device according to the present invention, comprising the step:
- Detecting the presence and / or integrity of the container, in particular an integrity of a closing element or breakable cap thereof, connected to the blood treatment device by means of at least one optical sensor and / or force sensor present on or associated with at least one connection element and / or at least one attachment element of the blood treatment device.

Furthermore, the method can comprise the step:
- aligning an inspection window present on the container, preferably an inspection window present in an outer tube of a connection element of the container, with an inspection window of the blood treatment device such that an optical sensor of the blood treatment device can optically inspect a closing element present on an inner tube of connection element of the container.

Further elements and / or features of the present invention can be described as follows: A container for concentrate according to the present disclosure can comprise at least one of the following:
- a hollow body for containing concentrate,
- a connection portion having at least one container connection element configured to interact with a corresponding machine connection element present on a blood treatment device to fluidically couple an inner volume of the hollow body to the blood treatment device,
- two container attachment elements arranged spaced apart from each other and configured to reversibly attach the container to two corresponding machine attachment elements present on a blood treatment device, wherein
- at least one of the two container attachment elements comprises a rounded and / or curved rotation surface configured to be placed onto a corresponding machine attachment element of the blood treatment device in an attachment position to be held there by gravity and configured to bulge in the direction of gravity in the attachment position.

The hollow body can have any desirable configuration suitable for containing a concentrate and can be a bag, cassette, cartouche, pouch or box. The hollow body can be formed of a flexible or rigid polymer material. The connection portion is preferably made of a rigid polymer material to ensure sufficient mechanical stability for connecting the container to a blood treatment device.

The rotation surface preferably is a surface configured to be placed onto a surface of a machine attachment element and configured to allow rotation of the connection portion of the container in the direction in that the container is attached to the blood treatment device. For example, the machine attachment element comprises a protruding pin and the container is attached to the blood treatment device by placing the rounded rotation surface of the container attachment element onto the protruding pin (also referred to as a stud). The container in this position orients itself and in particular its hollow body in a vertical direction, because the weight of the concentrate in the hollow body pulls downwards under the influence of gravity and the rotation surface moving on the protruding pin allows the connection portion to reflect the movement of the hollow body.

The rotation surface thus is preferably configured so that the circumference of a curvature defined by the rotation surface extends in the direction in that the container is moved to be attached to the blood treatment device. The rotation surface thus is preferably not configured so that the curvature defined by the rotation surface extends at an angle or at right angles to the direction in that the container is moved to be attached to the blood treatment device.

Preferably, the rotation surface is configured to allow a rocking motion, preferably back and forth along the direction of attaching the container to the blood treatment device, when the container attachment elements are placed with their rotation surface onto the corresponding machine attachment elements. A rotation surface as recited in the present disclosure thus preferably does not mean any curved or rounded surface capable of allowing any rotation, such as e.g. a rounded surface of a plug or socket of a plug connection, but preferably indicates a surface configured to allow a container attached to a blood treatment device by its attachment elements to adopt a vertical orientation on its own accord (e.g. without any human interaction) by rotating along the rotation surface under the influence of gravity.

The shape of the rotation surface can be chosen at will, as long as preferably a rounded and / or curved surface results that allows the connection portion of the container to rock back and forth with the rotation surface of the at least one container attachment element being placed on the at least one corresponding machine attachment element.

For example, the rounded and / or curved rotation surface can have a saddle shape or be formed as a half- cylinder or can have a shape between these two geometries. The ground surface of the half cylinder can be a circle, an oval or any other desirable shape, for example, a bean-shaped surface or a kidney-shaped surface.

The terms "rounded" and "curved" can be understood to mean any surface at least partially comprising a continuous curvature.

According to an embodiment, both of the two container attachment elements comprise a rounded and / or curved rotation surface. The two container attachment elements can be arranged at two opposing sides of the connection portion of the container.

At least one container attachment element can comprise one or more protrusions present at an end side of the rounded and / or curved rotation surface. When viewed in the attachment direction of a user attaching the container at a blood treatment device, the protrusion(s) are preferably arranged at a distal side of the rounded and / or curved rotation surface.

The one or more protrusions can project in a first direction and the container connection element can project in a second direction that is oriented at an angle, preferably essentially at right angles, to the first direction.

The one or more protrusions can also be configured to project essentially at right angles to the direction of gravity in a position in that the container attachment elements are attached to corresponding machine attachment elements.

Alternatively or additionally, the one or more protrusions project along a curved, preferably circular, circumference defined by the rounded and / or curved rotation surface.

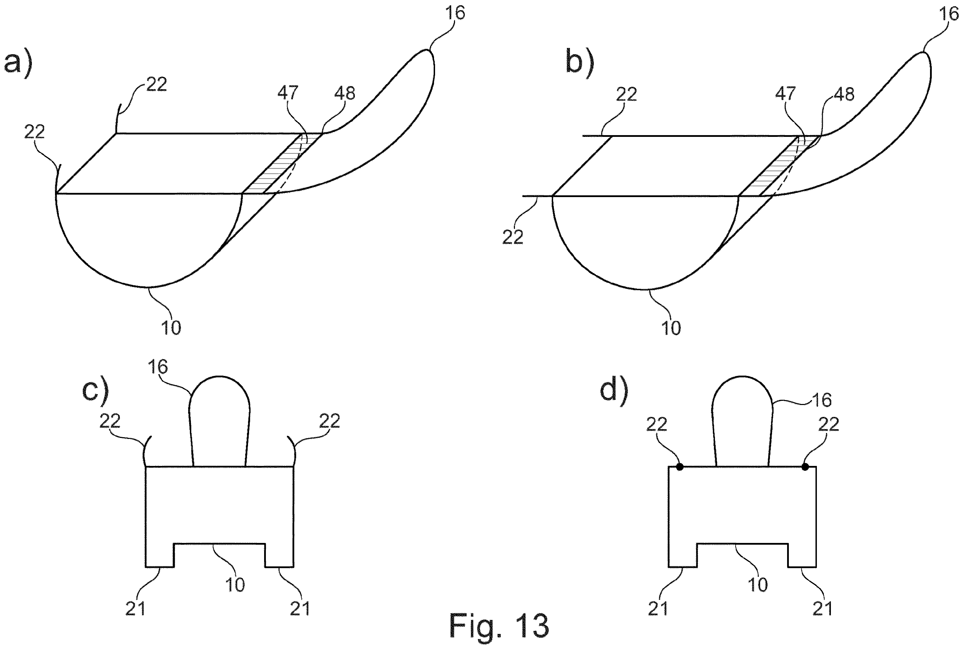

Exemplary arrangements of the protrusions relative to the rotation surface can be seen in Figs. 13 a) and 13b).

The protrusion(s) can serve the purpose of securing the container in the attachment position against an undesirable upwards movement in the vertical direction, e.g. upon a retraction of a machine connection element from the container connection element.

At the rounded and / or curved rotation surface of the container attachment element at least one rib can be present to prevent lateral movement of the container in the attachment position. The at least one rib preferably is arranged adjacent to the rounded and / or curved rotation surface and / or extends along the rounded and / or curved rotation surface.

At the rounded and / or curved rotation surface of the container attachment element two ribs can present that define a groove between them, wherein the groove is configured to at least partially receive one of the two corresponding machine attachment elements of the blood treatment device in the attachment position. The two ribs are preferably arranged at two opposing sides if the rotation surface.

When viewed in the attachment direction of a user attaching the container at a blood treatment device, the protrusion(s) are preferably arranged at a distal side of the rounded and / or curved rotation surface and a gripping portion at the user holds the container is preferably arranged at a proximal side of the rounded and / or curved rotation surface.

A linking portion can be present between the gripping portion and the rounded and / or curved rotation surface, so that a point linking the gripping portion to the container is offset from a center of gravity of the container.

When viewed against the attachment direction of a user attaching the container at a blood treatment device, the ribs are preferably arranged at the right and left sides of the rounded and / or curved rotation surface. This arrangement is illustrated e.g. in Fig. 13c) and 13d).

The groove between the two ribs can be solely defined by the two ribs and the rounded and / or curved rotation surface forming a bottom surface of the groove.

A longitudinal axis of the at least one container connection element can be oriented essentially in parallel to the longitudinal axis of the hollow body of the container. The at least one container connection element can have the form of a hollow cylinder opened at an end face of the connection portion, wherein a leading edge of the at least one container connection element comprises preferably a chamfered portion.

The at least one container connection element can comprise an outer lumen and an inner lumen preferably arranged concentrically to the outer lumen, wherein an outer surface of a wall defining the inner lumen has a polygonal, preferably a hexagonal, geometry. Preferably, the leading edge of the wall defining the inner lumen comprises a chamfered portion.

On the wall defining the inner lumen of the at least one connection element a closing element, preferably a cap, can be arranged, wherein an inner contour of the closing element arranged on the wall defining the inner lumen matches the polygonal, preferably hexagonal, geometry of the outer surface of the wall defining the inner tube.

The hexagonal configuration of the wall defining the inner lumen and the corresponding hexagonal configuration of the cap allows for a precise relative positioning of the cap and inner lumen. Preferably, the cap comprises six plates arranged adjacent to each other separated by intended breaking points and each plate is arranged on one edge of the hexagonal form of the wall defining the inner lumen. If pressure is exerted onto the plates, e.g. by a machine connection element, each plate pivots around the corresponding edge of the wall defining the inner lumen.

In order to facilitate the insertion of the machine connection element into the container connection element, the at least one container connection element can comprise at least one positioning element, preferably formed as a tooth in a leading edge of a wall defining the outer lumen of the container connection element.

The connection portion of the container can comprise a central opening for loading the hollow body with concentrate extending for a first length in a first direction and for a second length in a second direction, wherein the second length is smaller than the first length. For example, the central opening can have an oval, ellipsoid, super-elliptic, rectangular or figure-of-eight-shape or modifications of these shape, such as a rectangle with rounded corners.

According to an embodiment, two container connection elements and / or two container attachment elements are arranged at two opposing sides of the central opening so that the distance between the two container connection elements is reduced or minimized. In other words, the two container connection elements and / or two container attachment elements can be separated from each other over the second length rather than the first length.

To achieve a compact design of the connection portion of the container, in an area of juxtaposition of at least one container connection element and the central opening, the central opening can comprise a constriction portion convexly projecting into the opening of the central opening.

The constriction portion can comprise a concave section in that the connection element, preferably the outer lumen thereof, is arranged. Alternatively or additionally, a wall defining the central opening in the constriction portion at least partially forms a wall defining an outer lumen of at least one container connection element.

A blood treatment device according to the present disclosure and preferably configured to be used with a container according to the present disclosure, can comprise at least one of the following:
a housing,
two machine attachment elements stationarily arranged at the housing to that two corresponding container attachment elements of a container for concentrate are attachable, wherein at least one of the machine attachment elements comprises a holding element onto that a rounded rotation surface of a connector attachment element is to be placed in the attachment position of the container at the blood treatment device in that the container is held at the blood treatment device by gravity; and
two machine connection elements pivotably arranged at a flap pivotably arranged at the housing and each fluidically linked to a fluid path arranged in the flap, wherein the two machine connection elements are configured to fluidically couple the blood treatment device to the container for concentrate by each being inserted into a corresponding container connection element, wherein the two machine connection elements are rotationally movable in the same direction to be inserted into the corresponding container connection elements upon a pivoting movement of the flap.

At least one machine connection element can be movable along a first path into a treatment position to insert the at least one machine connection element into a corresponding container connection element and at the at least one machine connection element can be movable along a second path into a rinsing position to insert the at least one machine connection element into a corresponding rinsing element of the blood treatment device.

A blood treatment device according to the present invention can comprise a sensor configured to detect if a container has been attached to the blood treatment device in the attachment position and a control unit configured to move the at least one machine connection element into the treatment position if a container in the attachment position has been detected by the sensor.

At least one machine connection element can be pivotably arranged at a lever being rotatably arranged at the housing of the blood treatment device.

At least one machine connection element can comprise a wall with a serrated leading edge configured to open a closing element, preferably a cap, arranged on a wall defining an inner lumen of a container connection element by breaking intended breaking points present in the closing element.

The serrated leading edge can comprise one or more teeth, wherein in case of multiple teeth, each tooth preferably is arranged at the wall of the at least one machine connection element such that each tooth selectively exerts pressure onto a plate of the closing element upon insertion of the machine connection element into the container connection element, wherein the closing element can comprise multiple plates separated from each other via intended breaking points.

The two machine attachment elements can each comprise a holding element comprising a protrusion, preferably formed as a stud, and / or a retaining element, preferably formed as a ridge or rip, arranged at a distance from the protrusion and configured to retain a container attached to the blood treatment device in the attachment position against a vertically upward movement, in particular when the machine connection element is retracted from the container connection element.

A blood treatment device according to the present invention can comprise at least one rinsing element for receiving the at least one machine connection element in the rinsing position, wherein the rinsing element preferably is formed as a trough arranged inclined at an angle to a vertical direction, preferably at an angle of inclination of 40° to 50°, wherein preferably a fluid line is connected to the rinsing element in a lower most section of the rinsing element in the vertical direction.

The fluid line can be connected to the rinsing element in a peripheral area of a bottom surface of the rinsing element and / or can project at an angle of inclination of 40° to 50° relative to the bottom surface of the rinsing element.

The at least one rinsing element can be arranged stationary relative to a housing of the blood treatment device.

In other words, a blood treatment device according to the present disclosure is preferably configured to be used with a container according to the present invention, and can comprise at least one of the following:
- a housing,
- two machine attachment elements to that two corresponding container attachment elements of a container for concentrate are attachable, wherein at least one of the machine attachment elements comprises a holding element, e.g. in the shape of a pin or stud, onto that a rounded and / or curved rotation surface of a connector attachment element is to be placed in the attachment position of the container at the blood treatment device in that the container is held at the blood treatment device by gravity; and
- at least one machine connection element configured to fluidically couple the blood treatment device to the container for concentrate by interacting with a corresponding container connection element.

The at least one machine connection element is preferably movable along a first path into a treatment position to insert the at least one machine connection element into a corresponding container connection element and thereby fluidically couple the blood treatment device to the container.

Further, the at least one machine connection element is preferably movable along a second path into a rinsing position to insert the at least one machine connection element into a corresponding rinsing element of the blood treatment device.

The blood treatment device can comprise a sensor configured to detect if a container has been attached to the blood treatment device in the attachment position.

The sensor can be e.g. an optical sensor configured to inspect an area in that at least one container attachment element and / or at least one container connection element is arranged if a container is present in the attachment position.

Alternatively or additionally, the blood treatment device can be equipped with a mechanical sensor indicating the presence or absence of a container at the blood treatment device. For example, the mechanical sensor can have the form of a lever changing its relative position to the housing of the blood treatment device, e.g. by being depressed, when a container is attached to the blood treatment device. Thus, the relative position of the level indicates the presence or absence and / or correct attachment of the container to the blood treatment device.

The blood treatment device can further comprise a control unit configured to move the at least one machine connection element into the treatment position if a container in the attachment position has been detected by the sensor.

For this purpose, the control unit preferably is configured to operate an actuator such as a solenoid to move, e.g. push or pull, the connection element against the biasing force of a spring from the rinsing position into the treatment position.

In order to be movable between the treatment position and the rinsing position, the at least one machine connection element is preferably pivotably arranged at a lever being rotatably arranged at the housing of blood treatment device.

The at least one machine connection element can be pivotably arranged at a flap being rotatably arranged at the housing of blood treatment device. The flap is preferably movable by hand.

For example, the machine connection element can be moved in a rotatory path by moving the flap relative to the housing of the blood treatment device to juxtapose the machine connection element to the container connection element and then the machine connection element can be moved in a translational path to insert the connection element into the corresponding container connection element.

In order to reduce the peak amount of force required for opening a closing element closing the inner lumen of the container connection element, the at least one machine connection element can comprise a wall with a serrated leading edge configured to open a closing element, preferably a cap, arranged on a wall defining an inner lumen of a container connection element by breaking intended breaking points present in the closing element. The serrated leading edge can achieve a particularly targeted exertion of force onto the individual plates of the closing element.

The serrated leading edge can comprise one or more teeth, wherein in case of multiple teeth, each tooth preferably is arranged at the wall of the at least one machine connection element such that each tooth selectively exerts pressure onto a plate comprised by the closing element upon insertion of the machine connection element into the container connection element, wherein the closing element comprises multiple plates separated from each other via intended breaking points.

For example, the closing element comprises six plates and the serrated leading edge comprises three teeth that exert pressure onto three of the six plates.

The two machine attachment elements can each comprise a protrusion, preferably formed as a stud or pin, and a retaining element, preferably formed as a ridge or rip, arranged at a distance from the protrusion and configured to retain a container attached to the blood treatment device in the attachment position against a vertically upward movement, in particular when the machine connection elements are retracted from the container connection elements.

For example, if the container attachment elements are arranged on the machine attachment elements so that the rounded and / or curved rotation surfaces of the container attachment elements rest or sit on the studs or pins and the machine connection element inserted into the container connections elements is retracted upwards in vertical direction, friction may cause the container to follow the upward movement of the machine connection element. In this case, the retaining element limits the upward movement of the container to a position in that the container attachment element abuts the retaining element.

A blood treatment device according to the invention can further comprise at least one rinsing element for receiving the at least one machine connection element in the rinsing position, wherein the rinsing element preferably is formed as a trough that is arranged inclined at an angle to a vertical direction, preferably at an angle of inclination of 40° to 50°, wherein preferably a fluid line is connected to the rinsing element in a lower most section of the rinsing element in the vertical direction.

The inclined arrangement of the rinsing element relative to the vertical position can prevent encrustations forming in the rinsing element.

The fluid line can be connected to the rinsing element in a peripheral area of a bottom surface of the rinsing element and / or can project at an angle of inclination of 40° to 50° relative to the bottom surface of the rinsing element.

The inclined arrangement of the fluid line relative to the rinsing element relative can generate flow conditions in the rinsing element favorable for efficient rinsing.

The at least one rinsing element can be arranged stationary relative to a housing of the blood treatment device.

Another aspect of the present invention relates to a system comprising at least one container according to the present invention and at least one blood treatment device according to the present invention, wherein preferably the container is reversibly attached by gravity to the blood treatment device in the attachment position by means of the two container attachment elements interacting with the two machine attachment elements and / or the at least one machine connection element is fluidically coupled to the at least one container connection element.

In a system according to the present invention, preferably
- at least one container connection element comprises a wall defining an inner lumen, the wall having an outer surface with a hexagonal geometry, wherein a closing element is arranged on the wall defining the inner lumen and an inner contour of the closing element matches the hexagonal geometry of the outer surface of the wall defining the inner lumen of the container connection element, wherein the closing element comprises a closing portion comprising a plurality of plates arranged juxtaposed to each other separated by a plurality of intended breaking points; and
- at least one machine connection element comprises a wall with a serrated leading edge comprising a plurality of teeth, wherein
- the wall of the machine connection element and the closing element arranged on the wall defining the inner lumen of the container connection element are arranged in such a way that upon connection of the machine connection element with the container connection element, the teeth each exert pressure on one plate of the closing element so that each plate pivots around an edge of the hexagonal outer surface of the wall defining the inner lumen, thereby breaking at least one intended breaking point of the closing element and fluidically opening the closing element.

Yet another aspect of the present invention relates to a method for connecting a container for concentrate, preferably a container according to the present disclosure, to a blood treatment device, preferably a blood treatment device according to the present disclosure, comprising the step:
- reversibly attaching the container to the blood treatment device by affixing two container attachment elements to two corresponding machine attachment elements by a combination of a rotational movement and an axial movement of the container and / or the two container attachment elements relative to the blood treatment device and / or the two machine attachment elements, wherein the rotational movement and the axial movement are performed at least partially simultaneously.

It is to be noted that the present disclosure is not limited to the combinations of features explicitly disclosed therein, but comprises any other desirable combination of the disclosed features. In other words, features disclosed in the present application may be claimed in any desirable combination or in isolation.

Further features, advantages and technical effects of the present invention become apparent from the drawings showing preferred embodiments of the invention. The drawings merely serve purposes of illustrating the invention but do not purport to limit the invention. In the drawings, the same or similar components are denoted by the same reference signs.

In the drawings:
Fig. 1 illustrates how a container according to an embodiment of the invention is attached to a blood treatment device according to an embodiment of the invention;
Fig. 2 shows a top view of a connection portion of a container according to an embodiment of the invention;
Fig. 3 shows a bottom view of the connection portion of Fig. 2;
Fig. 4 shows a top view of a connection portion of a container according to another embodiment of the invention;
Fig. 5 illustrates the attachment elements of a blood treatment device according to an embodiment of the invention;
Fig. 6 illustrates a state in that a container according to the present invention is attached to a blood treatment device according to an embodiment of the invention and the connection elements of the blood treatment device are inserted into the connection elements of the container;
Fig. 7 a) shows a blood treatment device according to an embodiment of the invention in a treatment position;
Fig. 7b) shows the blood treatment device of Fig. 7a) in a rinsing position;
Fig. 8a) illustrates how the connection elements of the blood treatment device are moved between the treatment position and the rinsing position;
Fig. 8b) further illustrates how the connection elements of the blood treatment device are moved between the treatment position and the rinsing position;
Fig. 9a) shows a top view of a rinsing trough to be used with a blood treatment device according to the present invention;
Fig. 9b) shows a sectional view of the rinsing trough of Fig. 9a,
Fig. 10 shows a connection element of a container comprising an inspection window that is aligned with an inspection window of the blood treatment device;
Fig. 11 illustrates the use of strain gauges to detect the presence of a container attached to the blood treatment device and to determine a type of container; and
Fig. 12 illustrates to position of strain gauges at the connection elements, more precisely a lever thereof, of the blood treatment device.
Fig. 13a) illustrates a schematic overview of a rotation surface adjacent to that protrusions are arranged that follow the circumference defined by the curvature of the rotation surface;
Fig. 13b) illustrates a schematic overview of a rotation surface adjacent to that protrusions are arranged that project at right angles to the direction of gravity when the rotation surface is placed onto a machine attachment element in an attachment position;
Fig. 13 c) illustrates a view of the arrangement of Fig. 13a) when viewed from the left side in Fig. 14a) in that the two ribs are visible; and
Fig. 13d) illustrates a view of the arrangement of Fig. 13b) when viewed from the left side in Fig. 13b) in that the two ribs are visible.

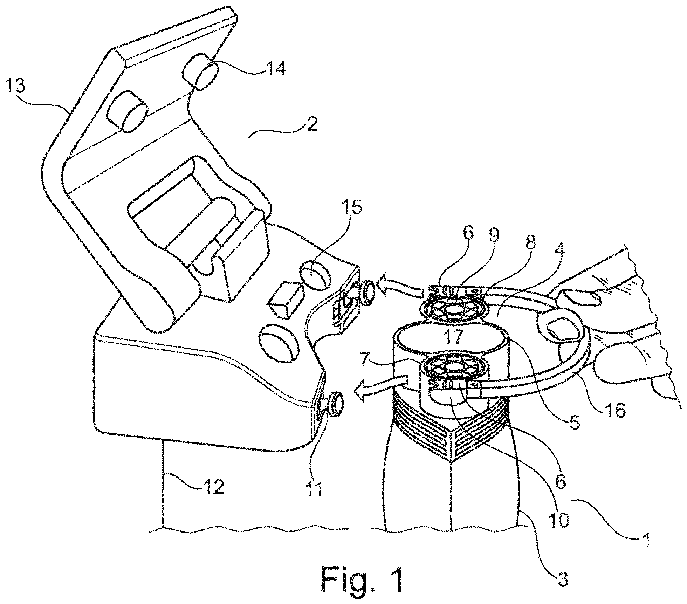

Fig. 1 illustrates how a container 1 according to an embodiment of the invention is attached to a blood treatment 2 device according to an embodiment of the invention.

The container 1 comprises a main body 3 for containing concentrate and a connection portion 4 having a flat end surface 5.

The container 1 comprises two attachment elements 6 arranged at two opposing sides of the connection portion 5 and two connection elements 7 arranged on opposite sides of a central opening 17 and each comprising an outer tube 8 and an inner tube that is closed by a cap 9 comprising multiple plates arranged similarly to the petals of a flower.

The two attachment elements 6 of the container 1 each comprise a rounded rotation surface 10 bulging in a longitudinal direction of the connection elements 7 and the main body 3 and configured to be placed onto a corresponding attachment element 11 of the blood treatment device 2 to be held there by gravity.

The attachment elements 11 of the blood treatment device preferably have the shape of studs.

The blood treatment device 2 comprises a main body 12 and a movable flap 13 that is pivotably arranged at the main body. The flap 14 comprises two connection elements 14 that can be inserted into the connection elements 7 (treatment position) of the container 1 or into two rinsing elements 15 of the blood treatment device (rinsing position).

As shown in Fig. 1, in order to attach the container 1 to the blood treatment device 2, a user grips the container at a gripping portion 16 and moves the container towards the blood treatment device with a combined axial and rotational movement as indicated by the arrows in Fig. 1.

The blood treatment device can comprise a control unit and a sensor for detecting a container 1 attached to the blood treatment device and if a container has been detected, the control unit can move the flap 13 and / or connection elements 14 from the rinsing position into the treatment position.

In the following section, the connection portion of a container according to the present invention is described in more detail.

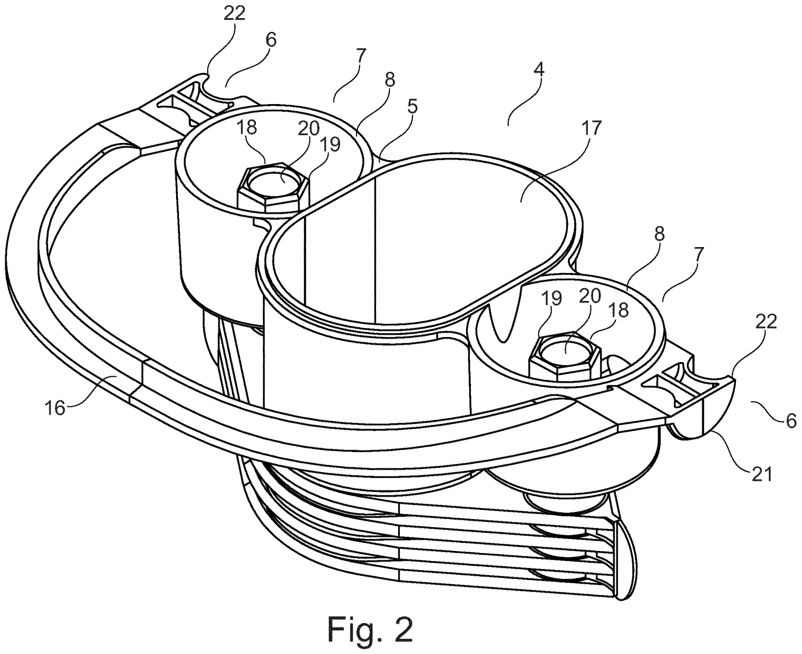

Fig. 2 shows a top view of a connection portion 4 of a container according to an embodiment of the invention.

In Fig. 2 it can be seen that the connection elements 7 of the container 1 each comprise an outer tube 8 and an inner tube 18 arranged concentrically to the outer tube 8. Each inner tube 18 comprises an outer surface or circumference 19 that has a hexagonal geometry and an inner surface or circumference 20 that has a circular geometry. In other words, the inner lumen of the inner tube 18 has a cylindrical shape.

The central opening 17 has an approximately oval or elliptical shape and the two connection elements 7 are arranged at the two opposing sides of the central opening 17 that are closest together.

The attachment elements 6 in this embodiment comprise two ribs 21 that define a groove between into that a stud 11 of a blood treatment element can be at least partially inserted.

Each attachment element 6 comprises a protrusion 22 arranged at a side of the rotation surface opposite to the gripping portion 16. The protrusions 22 are configured to interact with two corresponding protrusions of the blood treatment device acting as retaining elements in order to retain the container 1 in position upon retraction of the connection elements 14 of the blood treatment device form the connection elements 7 of the container.

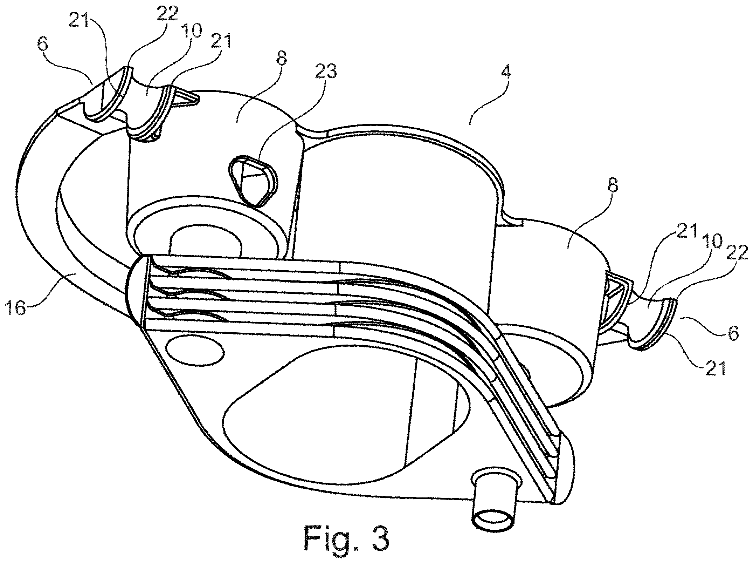

Fig. 3 shows a bottom view of the connection portion 4 of Fig. 2. In this view, the structure of the attachment elements 6 can be seen.

Each attachment element 6 comprises a rounded rotation surface 10 bulging convexly in a longitudinal direction of the connection elements 7 or outer tubes 8. On two opposing sides of each rotation surface 10 two rips 21 are arranged that extend along the rounded rotation surface 10. The two ribs 21 and the rotation surface 10 define a groove between them, similar to a poulie.

If the attachment elements 6 are placed onto the studs 11, the studs are inserted into the groove so that each rounded rotation surface 10 rests on a stud 11. The container can in this position rotate on the rotation surface 10 and rock and slide on the studs 11 while the rips 21 provide lateral stabilization.

Furthermore, in Fig. 3 an inspection window 23 present in at least one outer tube 8 can be seen. This inspection window 23 allows a sensor of the blood treatment device, e.g. an optical sensor, to inspect e.g. a closing element or cap present on an inner tube of 18 of the container 1 attached to the blood treatment device for integrity.

As shown in Fig. 3, the opening in the wall forming the inspection window 23 can have a triangular shape. This shape is particularly suitable if the blood treatment device comprises an optical sensor and two light sources, such as LEDs, that are arranged to form a triangle. Thus, the triangular shape of the inspection window preferably matches the triangular arrangement of the sensor and light source(s). This arrangement allows for an optimal illumination while reducing the overall size of the inspection window. Reducing the overall size of the inspection window increases the overall stability of the wall in that the inspection window is present.

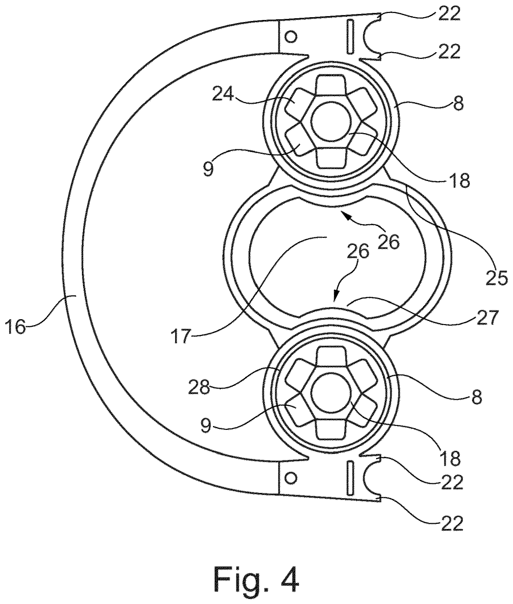

Fig. 4 shows a top view of a connection portion 4 of a container according to another embodiment of the invention. In this view, the two caps 9 used for closing the inner tube 18 of the connection elements 7 of the container 1 have opened / broken by the connection elements 14 of the blood treatment device exerting pressure onto the plates 24 of the caps 9. For this reason, in the view in Fig. 4 the inner tubes 18 are visible as opposed to the view in Fig. 1.

In the embodiment of Fig. 4 the wall 25 defining the central opening 17 has a slight hourglass contour and comprises a construction portion 26 indicated by the arrows in Fig. 4. The constriction portion 26 comprises a convex section 27 projecting into the central opening and a corresponding concave section 28 into that the outer tube 8 of the connection elements 7 are nestled.

This arrangement allows for larger diameter outer tubes 8 while keeping the dimension of the connection portion between the attachment elements 6 compact.

In the following section, selected features of a blood treatment device according to the present invention are described in more detail.

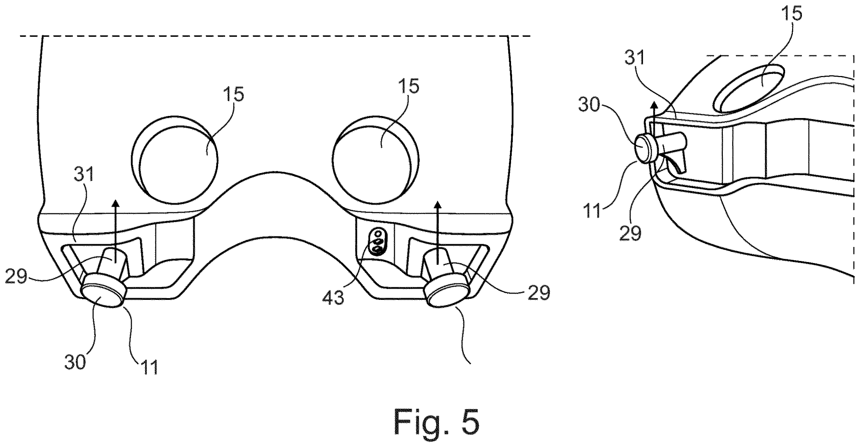

Fig. 5 illustrates the attachment elements 11 of a blood treatment device 2 according to an embodiment of the invention. In this embodiment, the studs comprise an elongated neck 29 that is configured to be at least partially inserted into a groove defined between the two ribs 21 of the attachment elements of a container 1, and a stopper 30 stopping the container 1 from sliding off the studs 11.

Adjacent to each stud 11 in vertical direction upwards and / or displaced from each stud in the direction of the rinsing elements 15 the blood treatment device comprises a ridge 31 configured to prevent an upward movement (as indicated by the arrows in Fig. 5) in the vertical direction of the container upon retraction of the connection elements 14 from the connection elements 7.

As the connection elements 14 are retracted upwards in vertical direction from the connection elements 7, the protrusions 22 of the attachment elements 6 of the container 1 abut the ridges 31 of the blood treatment device and thereby limit the upward movement of the container.

In the embodiment shown in Fig. 5 each ridge 31 at least partially surrounds each stud 11, for example on three sides.

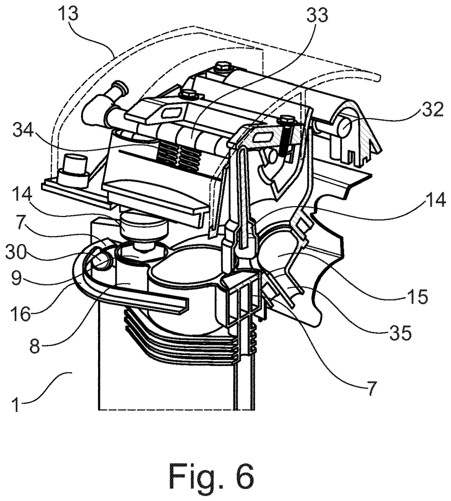

Fig. 6 illustrates a state in that a container 1 according to the present invention is attached to a blood treatment device 2 according to an embodiment of the invention and the connection elements 14 of the blood treatment device 2 are in the process of being inserted into the connection elements 7 of the container 1.

As can be seen in Fig. 6, the flap 13 and thus the connection elements 14 mounted thereon are arranged pivotably around a first pivot axis 32 at the blood treatment device.

Furthermore, the connection elements 14 are arranged pivotably around a second pivot axis 33 at the flap 13.

A movement around the second pivot axis 33 allows to move the connection elements from a position for insertion into the connection elements 7 of the container 1 (treatment position; shown in Fig. 6) to a position for insertion into the rinsing elements 15 (rinsing position).

The blood treatment device 2 mostly remains in the rinsing position if no treatment is performed. An actuator, e.g. a solenoid, can be provided to selectively move the connection elements against the biasing force of the spring from the rinsing position into the treatment position.

In Fig. 6, the arrangement of the rinsing elements 15 at the blood treatment device 2 can also be seen. The rinsing elements 15 are arranged inclined relative to a vertical direction or to a direction defined by a side surface of the blood treatment device and a fluid line 35 is connected to each rinsing element 15 in the lower most section so that fluid gathering in the rinsing elements can be removed.

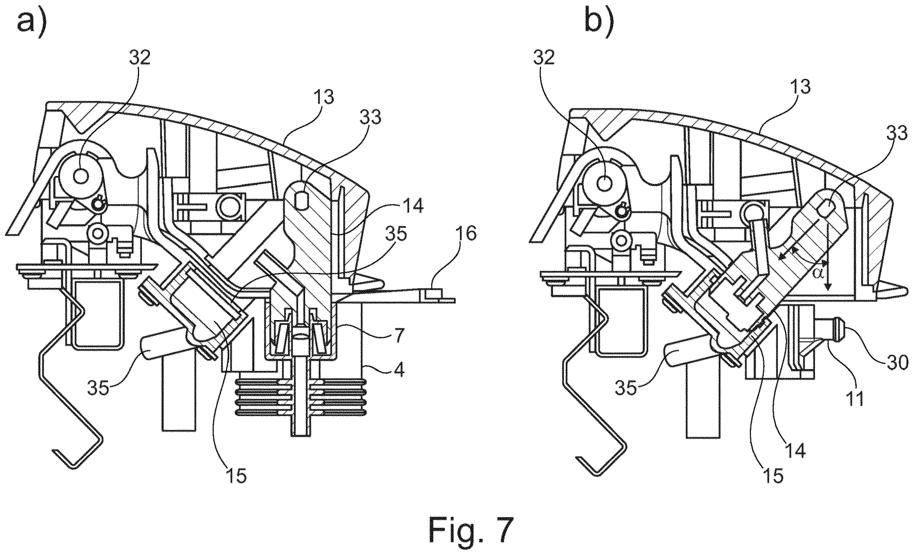

Fig. 7 a) shows a blood treatment device according to an embodiment of the invention in a treatment position. In this position, the flap 13 is closed, i.e. lowered towards the container 1 attached to the blood treatment device 2.

As shown in Fig. 7a, the connection elements 14 of the flap 13 are inserted into the connection elements 7 of the container 1. In this position, the rinsing elements 15 of the blood treatment device are covered or closed by a surface 36 of the flap 13. This prevents the rinsing elements 15 from being contaminated.

As shown in Fig. 7a, the connection elements 14 are arranged in this this position in an axial direction parallel to the vertical direction. In other words, the connection elements 14 in Fig. 7a project along the vertical direction downwards into the connection elements 7 of the container.

Fig. 7b) shows the blood treatment device of Fig. 7a) in a rinsing position. In this position, the connection elements 14 are rotated for an angle a shown in Fig. 7b) around the axis 33 from the treatment position shown in Fig. 7a) and are inserted in the rinsing elements 15 of the blood treatment device. The angle a shown in Fig. 7b) corresponds in this example to 43°.

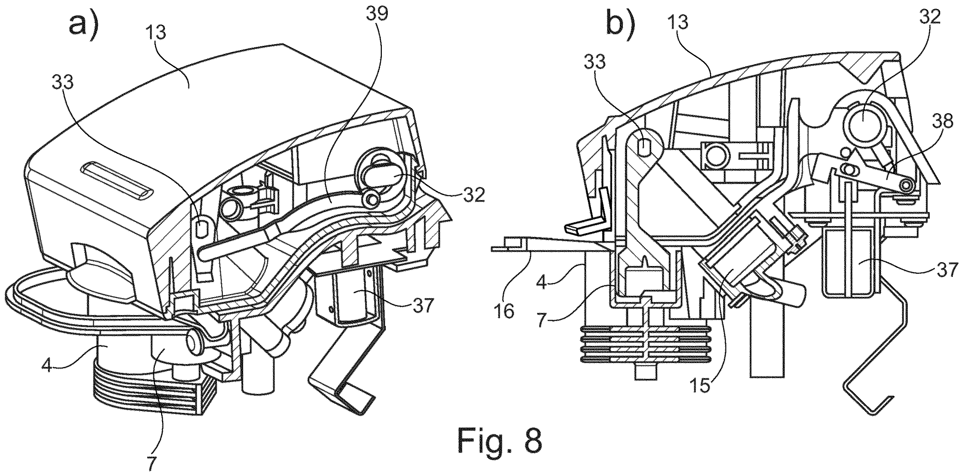

Figs. 8a) and 8b) illustrate how the connection elements 14 of the blood treatment device are moved between the treatment position and the rinsing position.

For this purpose, the blood treatment device 2 is equipped with a solenoid 37 that is configured to actuate a lever 38 that acts on the connection elements 14 via the lever 39 to push the connection elements 19 into the treatment position against the biasing force of a spring not shown in Fig. 8. The solenoid 37 is preferably activated by the control unit of the blood treatment device upon detection of a container being connected to the blood treatment device by the sensor, e.g. the IR proximity sensor.

If the solenoid 37 is not active, the spring pulls the connection elements 14 back into their rinsing position.

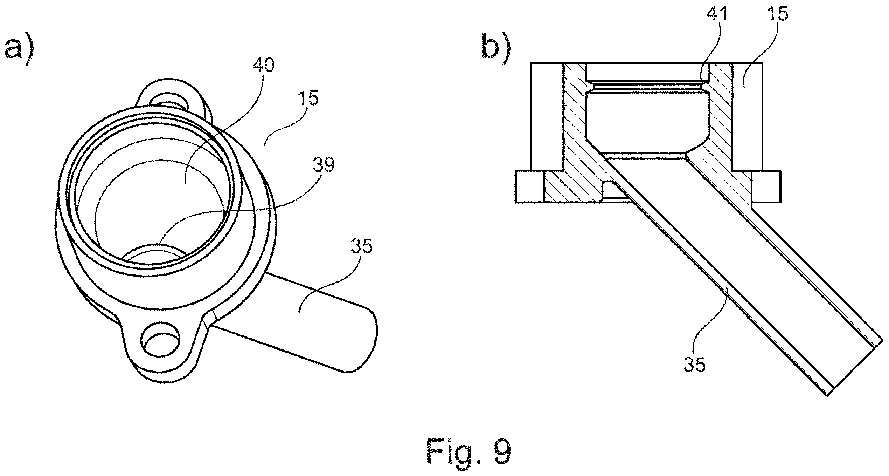

Fig. 9a) shows a top view of a rinsing element 15 or trough to be used with a blood treatment device 2 according to the present invention. The fluid line 25 is connected to the rinsing element 15 in a lower most section of the rinsing element 15.

In Fig. 9a, the rinsing elements 15 is shown in an inclined position similar to its mounting position at a blood treatment device according to the present invention.

The fluid line 35 is connected to the rinsing element 15 in a peripheral section of the bottom portion 40 of the rinsing element 15.

Fig. 9b) shows a sectional view of the rinsing element 15 of Fig. 9a. In this view, a circular sealing element 41 running along the inner circumference of the rinsing element 15 can be seen.

This sealing element can be e.g. an O-ring and serves the purpose of sealing a connection between the connection elements 14 of the blood treatment device 2 and the rinsing elements 15 in the rising position.

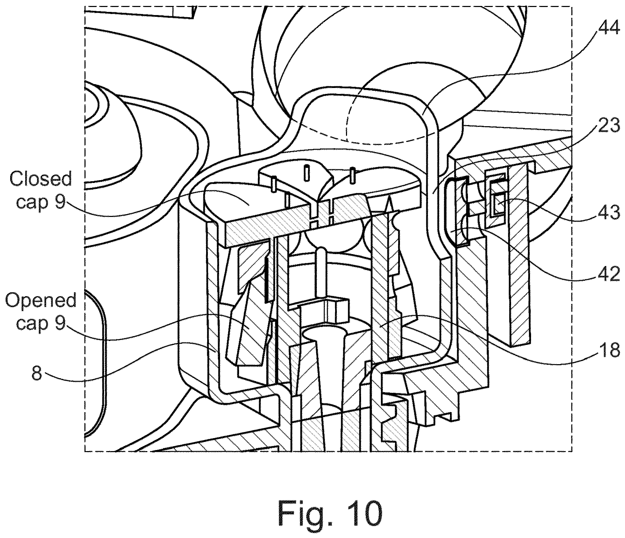

Fig. 10 shows a connection element 7 of a container comprising an outer tube 8 and an inner tube 18. On the inner tube 18 a cap 9 is arranged comprising multiple plates arranged similarly to the petals of a flower. In Fig. 10, the cap 9 is shown in two states, a closed state corresponding to an intact cap 9 and an opened or broken state corresponding to an opened cap 9.

The outer tube 8 comprises an inspection window 23 that can also be regarded as a cut or opening in the geometry of the disposable container.

As the container is attached to the blood treatment device, the outer surface of the outer tube 8 lies flush against an outer surface of the blood treatment device. In the outer surface of the blood treatment device there is an inspection window 42 that is aligned with the inspection window 23 of the container.

The optical sensor 43 thus has an unobstructed "view" of the closing element / cap 9 through the inspection windows 23 and 42. The field of inspection or field of vision of the sensor is indicated in Fig. 10 by dashed lines. The cap 9 is present in the field of inspection in the intact and opened state.

Also in Fig. 10 a positioning tooth 44 present at the leading edge of an outer tube 8 of the connection element can be seen.

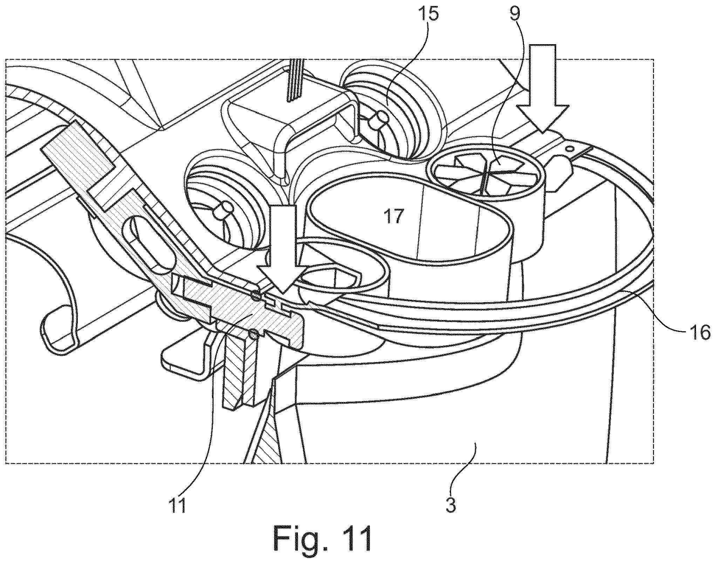

Fig. 11 illustrates the use of strain gauges to detect the presence of a container 1 atattached to the blood treatment device 2 and to determine a type of container. The studs 11 of the blood treatment device are each equipped with a strain gauge that measure the force (indicated by the arrows in Fig. 11) exerted by the weight of the container 1 attached to the studs 11.

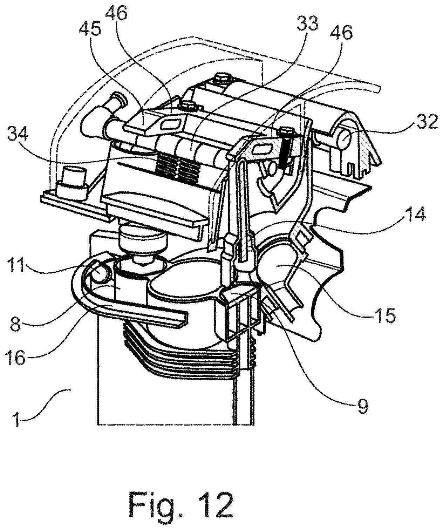

Fig. 12 illustrates to position of strain gauges 46 of the blood treatment device configured to detect a force required to be exerted by the connection elements 14 of the blood treatment device to open a cap 9 of the container 1 and connect the blood treatment device to the container.

The strain gauges 46 are present on two opposing sides of a lever 45 that connects the pivot axes 32 and 33.

Figs 13a) to Fig. 13d) show schematic illustrations of container attachment elements comprising rotation surfaces 10 according to the present disclosure.

In the embodiments of Figs 13a) and Fig. 13b) no ribs 21 are shown and in the embodiments of Figs 13c) and Fig. 13d) ribs 21 are shown.

Fig. 13a) illustrates a schematic overview of a rotation surface 10 adjacent to that protrusions 22 are arranged that follow the circumference defined by the curvature of the rotation surface. The two protrusions 22 are arranged relative to each other at two opposing sides of the rotation surface 10 (front and back side in Fig. 13a)).

At a side of the rotation surface 10 (right side in Fig. 13a)) opposite to the protrusions 22 (that are arranged at the left side in Fig. 13a)), a linking portion 47 is present linking the rotation surface 10 to the gripping portion 16. A linking point or connection point 48 in that the gripping portion 16 is joined to the linking portion 47 is offset from a center of gravity of the connection portion of the container.

Fig. 13b) illustrates a schematic overview of a rotation surface 10 adjacent to that protrusions 22 are arranged that project at right angles to the direction of gravity when the rotation surface 10 is placed onto a machine attachment element, e.g. a stud 11, in an attachment position.

Fig. 13c) illustrates a view of the arrangement of Fig. 13a) when viewed from the left side in Fig. 13a) in that the two ribs 21 are visible. The two ribs 21 are arranged at two opposing sides (left and right side in Fig. 13c)) of the rotation surface 10. The rotation surface 10 thus forms the bottom surface of a groove defined between the two ribs 21.

Fig. 13d) illustrates a view of the arrangement of Fig. 13b) when viewed from the left side in Fig. 13b) in that the two ribs 21 are visible. The two ribs 21 are arranged at two opposing sides (left and right side in Fig. 13d)) of the rotation surface 10. The rotation surface 10 thus forms the bottom surface of a groove defined between the two ribs 21.

## Claims

1. Blood treatment device, comprising
- a housing,
- a connection means configured to fluidically and / or mechanically connect the blood treatment device to a container, wherein the container comprises a closing element fluidically closing the container against the outside, and
- at least one sensor configured to detect the presence of a container connected to the connection means and further configured to detect an integrity status of the closing element of the container.

2. Blood treatment device according to claim 1, wherein the sensor is an optical sensor and the blood treatment device further comprises at least one inspection window configured to allow the optical sensor a field of inspection of the closing element of the container, in a position in that the container is connected to the blood treatment device, wherein the inspection window preferably comprises a transparent section preferably comprising a polymer material.

3. Blood treatment device according to claim 1 or 2, wherein the blood treatment device further comprises at least one illumination window configured to allow a light source of the blood treatment device to illuminate the closing element of the container, in a position in that the container is connected to the blood treatment device, wherein the illumination window preferably is arranged adjacent to the inspection window and comprises a transparent section preferably comprising a polymer material.

4. Blood treatment device according to one of claims 2 or 3, wherein the inspection window is arranged such that the closing element of the container is at least partially present in the field of inspection in a state in that the closing element is intact and in a state in that the closing element is opened.

5. Blood treatment device according to one of the preceding claims, further comprising an evaluation unit configured to determine based on optical measurement data from the optical sensor a proportion of the field of vision in that the closing element of the container is present and derive therefrom a conclusion regarding the presence of the container at the blood treatment device and / or a conclusion regarding an integrity status of the closing element, wherein the integrity status is preferably selected from the group consisting of intact/closed, partially opened and opened.

6. Blood treatment device according to one of the preceding claims, wherein the optical sensor is a colour sensor configured to measure optical intensities in at least three channels and the blood treatment device preferably comprises an evaluation unit configured to determine a colour of a closing element of a container.

7. Blood treatment device according to claim 6 comprising a memory in that optical characteristics, in particular characteristic optical intensities in at least three channels, of different types of containers connectable to the blood treatment device are stored, wherein the evaluation unit is configured to retrieve from the memory based on a determined colour of the closing element of the container a corresponding type of the container, in particular a corresponding size or volume or amount of content contained in the container.

8. Blood treatment device according to claim 7, wherein in the memory for each type of container at least one set of upper and lower limit values for each channel of the at least three channels is stored, wherein preferably or each type of container one set of upper and lower limit values for each of the at least three channels corresponding to an intact closing element and / or one set of upper and lower limit values for each of the at least three channels corresponding to an opened closing element and / or one set of upper and lower limit values for each of the at least three channels corresponding to container without a closing element are stored.

9. Blood treatment device according to one of the preceding claims, wherein the at least one sensor is a force sensor, preferably a strain gauge, configured to measure a force required to break a closing element present at a container upon connection of the container to the blood treatment device and / or configured to measure a force exerted by a container connected to the connections means od the blood treatment device.

10. Container for concentrate, comprising:
- a hollow body for containing concentrate,
- a container connection element configured to fluidically and / or mechanically connect the container to a blood treatment device, wherein
- the container connection element comprises an inspection window configured to allow an optical sensor of a blood treatment device in a field of inspection an optical inspection of a closing element, in particular a cap, fluidically closing the container, in an position in that the container is connected to the blood treatment device.

11. Container according to claim 10, wherein the connection element comprises an outer tube and an inner tube preferably arranged concentrically to the outer tube, wherein the inspection window is formed as an opening in the outer tube and / or an outer surface of the inner tube has a polygonal, preferably a hexagonal, geometry.

12. Container according to claim 11, wherein on the inner tube of the connection element a closing element, preferably a cap, is arranged and the inspection window is arranged so that the closing element is at least partially in the field of inspection in a state in that the closing element is intact and / or in a state in that the closing element is opened, wherein preferably an inner contour of the closing element arranged on the inner tube matches the polygonal, preferably hexagonal, geometry of the outer surface of the inner tube.

13. System comprising at least one blood treatment device, preferably a blood treatment device according to one of claims 1 to 9, and at least one container, preferably a container according to one of claims 10 to 12, wherein the container is reversibly connectable to the blood treatment device by means of a container connection element present on the container interacting with connection means present on the blood treatment device in a position in that the inspection window of the container and the inspection window of the blood treatment device are at least partially aligned to allow at least one optical sensor of the blood treatment device to optically inspect at least one closing element of the container.

14. Method for connecting a container for concentrate, preferably a container according of one of claims 10 to 12, to a blood treatment device, preferably a blood treatment device according to one of claims 1 to 9, comprising the step:
- Detecting the presence and / or integrity of the container, in particular a closing element or breakable cap thereof, connected to the blood treatment device by means of at least one optical sensor and / or force sensor present on or associated with the connections means of the blood treatment device.
